# EUROPEAN PATENT SPECIFICATION

(11) **EP 2 558 097 B1**
(45) Date of publication and mention of the grant of the patent: **25.10.2017**
(21) Application number: 11768285.6
(22) Date of filing: 14.04.2011
(51) Int. Cl.: A61K 31/501, A61P 11/06, A61P 31/16, A61K 45/06

(54) **Vapendavir for the alleviation of symptoms of asthma in a subject having a HRV infection**
Vapendavir zur Linderung der Symptome von Asthma in Patienten mit einer HRV-Infektion
Vapendavir pour l'atténuation des symptômes de l'asthme chez un sujet ayant une infection à RVH

(30) Priority: 15.04.2010 AU 2010901601
(43) Date of publication of application: 20.02.2013
(73) Proprietor: Biota Scientific Management Pty Ltd, Notting Hill, VIC 3168 (AU)
(72) Inventor: LAMBERT, John Nicholas, Notting Hill, Victoria 3168 (AU); RYAN, Jane, Notting Hill, Victoria 3168 (AU); WILSON, Janet Marie, Notting Hill, Victoria 3168 (AU)
(74) Representative: Weickmann & Weickmann PartmbB
(86) International application number: PCT/AU2011/000434
(87) International publication number: WO 2011/127538

(56) References cited:
- WO-A1-02/50045
- WO-A1-2009/143571
- WO-A1-2010/009288
- ROHDE G: "Drug targets in rhinoviral infections", INFECTIOUS DISORDERS - DRUG TARGETS 2009 BENTHAM SCIENCE PUBLISHERS B.V. NLD, vol. 9, no. 2, 2009, pages 126-132, XP009171714, ISSN: 1871-5265
- ARMANDO M. DE PALMA ET AL: "Selective inhibitors of picornavirus replication", MEDICINAL RESEARCH REVIEWS, vol. 28, no. 6, 1 November 2008 (2008-11-01), pages 823-884, XP055074067, ISSN: 0198-6325, DOI: 10.1002/med.20125
- MALLIA, P. ET AL.: 'Exacerbations of Asthma and Chronic Obstructive Pulmonary Disease (COPD): Focus on Virus Induced Exacerbations' CURRENT PHARMACEUTICAL DESIGN vol. 13, 2007, pages 73 - 97
- GERN ET AL.: 'Association of Rhinovirus Infections with Asthma' CLINICAL MICROBIOLOGY REVIEWS vol. 12, no. 1, 1999, pages 9 - 18

## Description

### FIELD OF THE INVENTION

The present invention relates to the alleviation of symptoms of asthma.

### BACKGROUND OF THE INVENTION

Asthma is a chronic disease of the bronchial airways. It is believed that at least 300 million people are affected by asthma worldwide. The symptoms of asthma are caused by the inflammation and reversible constriction of airways and range from wheezing to breathlessness to life-threatening asthma attacks.

The prevalence of asthma is believed to have doubled in the last 30 years. Examples of the economic impact of the disease include absenteeism from school or work and an increasing reliance on the health system. Although asthma affects people of all ages and ethnicities, it is considered to be the most common chronic disease suffered by children. In the year 2000 it was estimated that over 5.3 million children under the age of 18 suffered from asthma in the United States alone and the rate of asthma in children under the age of 5 had increased by 160% in the preceding 15 years. There is no present reason to expect that this trend will abate in the future and numerous trials for treatment are in progress.

The underlying mechanisms of the pathogenesis of asthma, though extensively investigated, are complex and not well understood. Airway inflammation, airway hyperresponsiveness and airway remodelling may be considered to be the general underlying pathogenic features of asthma. More specific pathogenic features of asthma are currently understood to include the following: production of IgE, airway smooth muscle (ASM) and goblet cell hypertrophy/hyperplasia, mucus hypersecretion, eosinophil, neutrophil and mononuclear cell infiltration into the submucosal layer of airways, mast cell and macrophage activation, sloughing of airway epithelial cells and the release of a range of mediators from Th2 cells and activated inflammatory cells that damage the mucosal epithelial lining and promote exaggerated repair responses (Hansbro, N.G., et. al., Pharmacology & Therapeutics (2008) 117:313-353).

There is no cure for asthma. There are however drug therapies available to assist sufferers in managing asthma, particularly the symptoms and exacerbations of the underlying or pre-existing condition. The drug therapies which are currently available include: bronchodilators such as theophylline, anticholinergics (such as ipratropium), short-acting selective ß2-adrenergic receptor agonists (such as levalbuterol, pirbuterol and albuterol also known as salbutamol), inhaled long-acting ß2-adrenergic receptor agonists (LABA) (such as salmeterol and formoterol), leukotriene modifiers or leukotriene receptor antagonists (LTRA; such as montelukast, pranlukast, zafirlukast and zileuton); inhaled corticosteroids (such as fluticasone, budesonide, trimcinolone, flunisolide, beclomethasone, mometasone and ciclesonide); and oral/intravenous corticosteroids (such as prednisone and methylprednisolone) which are reserved for the treatment of severe asthmatic episodes due to serious side effects. Inhaled corticosteroids may be combined with long acting β-adrenergic receptor agonists or short acting β-adrenergic receptor agonists.. Omalizumab, an anti-IgE monoclonal antibody, may be administered subcutaneously in cases where inhaled corticosteroids, long-acting β-adrenergic receptor agonists and leukotriene modifiers are ineffective or must be avoided due to adverse effects. However, omalizumab is a comparatively costly course of treatment (Fanta, C.H., New Eng. J. Med (2009) 360(10):1002-1014).

Furthermore, the mode of delivery of these asthmatic medications is important, and inhaled or aerosol delivery of corticosteroids. for example, is preferred as it maximizes the local effects of the drug in the lungs and minimizes systemic side effects when compared with oral therapy (Takizawa, H., Recent Patents on Inflammation & Allergy Drug Discovery (2009) 3(3):232-239 and Fanta, C.H., New Eng. J. Med (2009) 360(10):1002-1014),

However the response to asthmatic medications is variable amongst sufferers. It is believed that one or more of these classes of medications is of no therapeutic benefit in up to 50% of asthmatics. Serious adverse effects have also been associated with various asthma medications (Duan, Q.L. and Tantisira, K.G., Current Pharmaceutical Design (2009) 15(32):3742-3753).

As there is currently no cure for asthma it is recommended that sufferers manage these underlying or pre-existing conditions by avoiding certain events or risk factors which may trigger exacerbations of their symptoms. The triggers for an asthmatic episode are varied and include exercise, cold air, pollutants, irritants, food allergies, allergens and bacterial and viral respiratory infections.

Exposure to certain triggers such as food allergies, irritants such as smoke and allergens may be minimised by sufferer awareness and/or changes in lifestyle. However other triggers such as bacterial or viral respiratory infection are more difficult to avoid given the ease with which they may be transmitted in a population.

A number of respiratory viral infections have been studied with respect to asthma. A number of irriportant factors have been postulated as being released by the epithelium in the case of viral infection and include: cytokines (interferons (IFN-α, IFN-β, IFN-λ), interleukins (IL-1b, IL-6, IL-8, IL-10, IL-11, IL-16) and tumour necrosis factor (TNF-α)), chemokines (IL-8, monocyte chemoattractant proteins (MCP-1, MCP-4), macrophage inhibitory proteins (MIP-1α, MIP-3α, regulated on activation normal T cell expressed and secreted (RANTES) and eotaxins (1, 2, epithelial neutrophil-activating peptide-78 (ENA-78), IFN-γ inducible protein-10 (IP-10)); major histocompatibility (MHC) molecules (cellular adhesion molecules (MHC I, MHC II)); adhesion molecules (ICAM-1, VCAM-1, Ep-CAM); integrins (α1-6, 8, 9); pattern recognition receptors (toll-like receptors (b1, 4-6, 8, TLRs 1-10)); lipid mediators (prostaglandins (CD14, PGE₂, PGF₂ₓ), leukotrienes (thromboxane B₂, LTB₄, LTC₄, LTD₄, LTE₄)); growth factors (epidermal GF, platelet derived GF, transforming GF (TGF)-α,β), basic fibroblast GF (bFGF), insulin-like GF) and colony stimulating factors (granulocyte CSF (G-CSF), granulocyte-macrophage CSF (GM-CSF)); antimicrobial peptides (defensins (α, β, lysozyme), collectins (lactoferrin, surfactant protein-A,D); neuropeptides (endothelins (substance P)); mucins (calcitonin gene-related peptide (CGRP)); oxygen radicals; and gases (nitric oxide) (Dakhama, A., et al.. J. Pediatr, Infect. Dis. (2005) 24 S159-S169 and Hansbro, N.G., et. al., Pharmacology & Therapeutics (2008) 117:313-353).

Among the number of respiratory viral infections that have been studied with respect to asthma is rhinoviral infection or human rhinovirus (HRV). HRV is considered to be the causative agent in an estimated 30 - 35 % of episodes of the common cold and it is estimated that one billion episodes of the common cold occur annually in the United States alone. However to date there are no approved therapies for the treatment of rhinovirus infection. Two potential HRV candidates, namely inhaled pirodavir and oral pleconaril have not progressed in the clinic due to lack of demonstrated benefit and US Food and Drug Administration (FDA) safety concerns (Rohde, G., Infectious Disorders - Drug Targets (2009) 9:126-132). As recently published in WO2010/009288, it was stated that "pleconaril administered orally did not have an effect on asthma exacerbation". Clinical trials have been conducted to assess the effect of pleconaril nasal spray in asthmatics, however at present the results of this trial have not been released (see the US government Clinical Trial Identifier No NCT/00394914 at http://www.clinicaltrials.gov/ct2/show/NCT00394914/).

Armando M. DePalma et al., Medicinal Research Reviews (2008), Vol. 28:6, 823-884 refers to a clinical trial, where Vapendavir is used in the treatment of HRV infections. Patients in this study are high-risk COPD and asthma patients, but the administration of Vapendavir clearly aims at the treatment of HRV infections. International patent application WO 02/50045 discloses a class of antiviral compounds, that were found to exhibit anti-picorna viral properties. The compounds described also include Vapendavir. However, it is not suggested that this compound might be suitable for alleviating asthma symptoms. Similarly, WO 2009/143571 discloses the bis-dihydrogenphosphate salt of Vapendavir for use in the treatment of picorna virus infections without providing any hint as to the effect on asthma symptoms

Asthma sufferers are at great risk of severe asthmatic episodes triggered by events such as the common cold. International patent application WO 2010/009288 describes the treatment or prevention of asthma exazerbations by orally inhaling or intranasally administering an anti-viral active pharmaceutical agent, in particular Pleconaril. Only with this specific form of administration, a significant effect upon asthma symptoms is observed. In contrast, orally administered Pleconaril such as oral tablets is described to have no effect on asthma exazerbations. Accordingly, there remains an ongoing need for efficacious medicaments to alleviate the symptoms of asthma that are suitable for oral administration.

### Summary of the invention

It has now been discovered, that a particular compound which is effective against HRV is useful in the alleviation of the symptoms of asthma.

Accordingly in one aspect there is provided 3-Ethoxy-6-{2-[1-(6-methyl-pyridazin-3-yl)-piperidin-4-yl]-ethoxy}-benzo[d]isoxazole or a pharmaceutically acceptable salt thereof for use in the alleviation of symptoms of asthma in a subject having a HRV infection wherein the symptoms of asthma are selected from the group consisting of reduced lung function, reduced lung volume, coughing, wheezing, breathlessness and airway necrosis and wherein 3-ethoxy-6-{2-[1-(6-methyl-pyridazin-3-yl)-piperidin-4-yl]-ethoxy}-benzo[d]isoxazole or the pharmaceutically acceptable salt thereof is formulated for oral administration.

The asthma sufferer has a HRV infection.

The administration is oral administration.

In a further embodiment the oral administration is oral enteral administration. In still another embodiment the orally administered 3-ethoxy-6-{2-[1-(6-methyl-pyridazin-3-yl)-piperidin-4-y)]-ethoxy}-benzo[d]isoxazole or a pharmaceutically acceptable salt thereof is in a solid form or a liquid form. In yet a further embodiment the solid form is a tablet or capsule.

In another embodiment the 3-ethoxy-6-{2-[1-(6-methyl-pyridazin-3-yl)-piperidin-4-yl]-ethoxy}-benzo[d]isoxazole or a pharmaceutically acceptable salt thereof is administered in a dosage amount of from 1 mg to 800 mg per day.

In yet another embodiment the 3-ethoxy-6-{2-[1-(6-methyl-pyridazin-3-yl)-piperidin-4-yl]-ethoxy}-benzo[d]isoxazole or a pharmaceutically acceptable salt thereof is administered separately, simultaneously or sequentially in combination with at least one asthma medication.

### BRIEF DESCRIPTION OF THE FIGURES

FIGURE 1: Bar chart showing HRV AUC viral load determined by culture ITT population (± 90 and 95% confidence intervals) for 3-Ethoxy-6-{2-[1-(6-methyl-pyridazin-3-yl)-piperidin-4-yl]-ethoxy}-benzo[d]isoxazole at 25mg, 100mg and 400mg as against placebo.
FIGURE 2: Bar chart showing preliminary results (n=1) for 3-Ethoxy-6-{2-[1-(6-methyl-pyridazin-3-yl)-piperidin-4-y)]-ethoxy}-benzo[d]isoxazole free base in Human cell-derived tissue culture model for epithelial tissue of the human respiratory tract (EpiAirway^{™} system) for tissue derived from normal donors, asthmatic donors and COPD donors.

### DETAILED DESCRIPTION OF THE INVENTION

3-Ethoxy-6-{2-[1-(6-methyl-pyridazin-3-yl)-piperidin-4-yl]-ethoxy}-benzo[d]isoxazole is disclosed in WO02/50045 (the entire contents of which is incorporated herein by reference) and has the following structure:

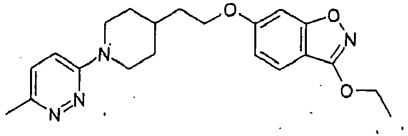

3-Ethoxy-6-{2-[1-(6-methyl-pyridazin-3-yl)-piperidin-4-yl]-ethoxy}-benzo[d]isoxazole has been shown to possess anti-HRV activity *in vitro* (IC₅₀ 0.001µg/ml with respect to HRV strain 2 and IC₅₀ 0.005µg/ml with respect of HRV strain 14). 3-ethoxy-6-{2-[1-(6-methyl-pyridazin-3-yl)-piperidin-4-yl]-ethoxy}-benzo[d]isoxazole and pharmaceutically acceptable salts thereof may now be shown to be effective in the alleviation of symptoms of asthma as defined in the claims.

Without wishing to be bound by theory it is believed that 3-ethoxy-6-{2-[1-(6-methyl-pyridazin-3-yl)-piperidin-4-yl]-ethoxy}-benzo[d]isoxazole and pharmaceutically acceptable salts thereof effectively target a viral trigger of symptoms of asthma j, the viral trigger having no approved therapy to date.

The asthma sufferer may be an adult (≥18 years of age) or a child (<18 years of age). In the case of a child asthma sufferer the asthma is typically referred to as paediatric asthma. The sufferer may be a mild, moderate or severe asthmatic (as assessed using the Global Initiative for Asthma "GINA" guidelines).

Due to the complex nature of respiratory conditions and diseases, such as asthma
there is an ongoing need for effective treatment and management strategies. Alternative strategies are therefore envisaged to complement the therapeutically active agents (and medicaments) currently prescribed to manage or prevent the symptoms in sufferers of respiratory conditions and diseases such as asthma sufferers (particularly children).

One such strategy may involve 3-ethoxy-6-{2-[1-(6-methyl-pyridazin-3-yl)-piperidin-4-yl)-ethoxy}-benzo[d]isoxazole or a pharmaceutically acceptable salt thereof being administered in combination with at least one asthma medication. In such a combination, the mode of administration may involve administering the 3-ethoxy-6-{2-[1-(6-methyl-pyridazin-3-yl)-piperidin-4-yl]-ethoxy}-benzo[d]isoxazole or a pharmaceutically acceptable salt thereof and the at least one asthma medication separately, simultaneously or sequentially in the same or separate dosage forms by the same or different administration routes.

Accordingly, an embodiment provides for administration of 3-ethoxy-6-{2-[1-(6-methyl-pyridazin-3-yl)-piperidin-4-yl)-ethoxy}-benzo[d]isoxazole or a pharmaceutically acceptable salt thereof in combination with at least one asthma medication.

Also described is a pharmaceutical combination comprising 3-ethoxy-6-{2-[1-(6-methyl-pyridazin-3-yl)-piperidin-4-yl]-ethoxy}-benzo[d]isoxazole or a pharmaceutically acceptable salt thereof and at least one asthma medication. Typically the pharmaceutical combination also comprises a pharmaceutically-acceptable adjuvant, diluent or carrier.

Examples of asthma medications include theophylline, anticholinergics (such as ipratropium), short-acting selective ß2-adrenergic receptor agonists (such as levalbuterol, pirbuterol and albuterol also known as salbutamol), inhaled long-acting ß2-adrenergic receptor agonists (LABA) (such as salmeterol and formoterol), leukotriene modifiers or leukotriene receptor antagonists (LTRA; such as montelukast, pranlukast, zafirlukast and zileuton); inhaled corticosteroids (such as fluticasone, budesonide, trimcinolone, flunisolide, beclomethasone, mometasone and ciclesonide); and oral/intravenous corticosteroids (such as prednisone and methylprednisolone). Bronchodilators and corticosteroids arc particularly preferred.

The described pharmaceutical combination may be a pharmaceutical composition wherein the 3-ethoxy-6-{2-[1-(6-methyl-pyridazin-3-yl)-piperidin-4-yl]-ethoxy}-benzo[d]isoxazole or a pharmaceutically acceptable salt thereof and the at least one asthmatic agent are in admixture. Typically the pharmaceutical composition also comprises a pharmaceutically-acceptable adjuvant, diluent or carrier.

The described pharmaceutical combination may be provided as a kit of parts of 3-ethoxy-6-{2-[1-(6-methyl-pyridazin-3-yl)-piperidin-4-yl]-ethoxy}-benzo[d]isoxazole or pharmaceutically acceptable salt thereof and the other asthmatic agent. In these aspects each component of the kit of parts is provided in a form that is suitable for administration in conjunction with the other component. In this respect the two components in the kit of parts may be: (i) provided as separate formulations (i. e. independently of one another), which are subsequently brought together for use in conjunction with each' other in combination therapy; or (ii) packaged and presented together as separate components of a combination pack for use in conjunction with each other in combination therapy. Typically each component of such a pharmaceutical combination also comprises a pharmaceutically-acceptable adjuvant, diluent or carrier.

According to the invention, 3-ethoxy-6-{2-[1-(6-methyl-pyridazin-3-yl)-piperidin-4-yl]-ethoxy}-benzo[d]isoxazole or a pharmaceutically acceptable salt thereof is formulated for oral delivery.

Compositions are provided in a form suitable for oral administration.

Oral compositions or formulations may be in a liquid or a solid form. Examples of such forms include tablets, capsules, suspensions, emulsions and syrups. Solid forms such as tables and capsules are particularly preferred.

Suitable solid form preparations may also include those which are intended to be converted, shortly before use, to liquid form preparations for oral administration.

3-Ethoxy-6-{2-[1-(6-methyl-pyridazin-3-yl)-piperidin-4-yl]-ethoxy}-benzo[d]isoxazole or a pharmaceutically acceptable salt thereof, may be formulated together with one or more pharmaceutically acceptable carriers, diluents and/or excipients.

Carriers and/or diluents include any and all solvents (including where used to form a solvate such as a hydrate), dispersion media, coatings, antibacterial and antifungal agents, isotonic and absorption delaying agents and the like. The use of such media and agents for pharmaceutical active substances is well known in the art. Carriers and excipients are ideally "pharmaceutically acceptable" meaning that the carrier or excipient is substantially compatible with the other ingredients of the composition or formulation and is substantially not deleterious to a subject. The active ingredient may be formulated, for example, by employing conventional solid or liquid vehicles or diluents, as well as pharmaceutical additives of a type appropriate to the mode of desired administration (for example, excipients, binders, preservatives, stabilizers, flavours, etc.) according to techniques such as those well known in the art of pharmaceutical formulation (See, for example, Remington: The Science and Practice of Pharmacy, 21st Ed., 2005. Lippincott Williams & Wilkins). Examples of suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like.

Suitable liquid form preparations include solutions, suspensions and emulsions, for example, water or water-propylene glycol solutions. Aqueous solutions suitable for oral use can be prepared by dissolving 3-ethoxy-6-{2-[1-(6-methyl-pyridazin-3-yl)-piperidin-4-yl]-ethoxy}-benzo[d]isoxazole or a pharmaceutically acceptable salt thereof in water and adding suitable colorants, flavours, stabilizing and thickening agents as desired. Aqueous suspensions suitable for oral use can be made by dispersing the finely divided active component in water with a viscous material, such as natural or synthetic gums, resins, methylcellulose, sodium carboxymethylcellulose or other well known suspending agents.

Solid form preparations include powders, tablets, pills, capsules, cachets, lozenges, suppositories, and dispensable granules. The term "preparation" is intended to include the formulation of 3-ethoxy-6-{2-[1-(6-methyl-pyridazin-3-yl)-piperidin-4-yl]-ethoxy}-benzo[d]isoxazole or a pharmaceutically acceptable salt thereof with encapsulating material as carrier, thereby providing a capsule in which the active component, with or without carriers, is surrounded by a carrier. In the form of a dry powder the preparation may be, for example, a mix of the compound in a suitable powder base such as glucose, lactose, starch, starch derivatives such as hydroxypropylmethyl cellulose and polyvinylpyrrolidone (PVP). Lactose is a preferred powder base. The powdered compound or composition may be presented in a unit dose form. In powders, the carrier is a finely divided solid that is in a mixture with the finely divided active component. In tablets, the active component is mixed with the carrier having the necessary binding capacity in suitable proportions and compacted in the shape and size desired. Preferred solid form preparations for oral administration are tablets, pills, lozenges and capsules, with tablets and capsules being particularly preferred.

In a unit dose form, the preparation is subdivided into unit doses containing appropriate quantities of 3-ethoxy-6-{2-[1-(6-methyl-pyridazin-3-yl)-piperidin-4-yl]-ethoxy}-benzo[d]isoxazole or a pharmaceutically acceptable salt thereof and in such form may be employed as solids, such as tablets or filled capsules, or liquids such as solutions, suspensions, emulsions, elixirs, or capsules filled with the same, all for oral use.

The unit dosage form can be a packaged preparation, the package containing discrete quantities of preparation such as tablets, capsules and powders in vials or ampoules. The unit dosage form can also be a capsule, table, cachet, or lozenge itself or it can be the appropriate number of any of these in packaged form. Such unit dosage forms may contain any suitable effective amount of the active ingredient commensurate with the intended daily dosage range to be employed. Formulations containing 0.1 to 1000 milligrams of active ingredient per dosage for provide representative unit dosage forms. In some embodiments the 3-ethoxy-6-{2-[1-(6-methyl-pyridazin-3-yl)-piperidin-4-yl]-ethoxy}-benzo[d]isoxazole or a pharmaceutically acceptable salt thereof is administered in a dosage amount of from 1mg to 800mg per day, from 1mg to 600mg per day, from 1mg to 400mg per day, 1mg to 200mg per day or from 1mg to 100mg per day. The dosage forms may comprise, as the active component, either a compound of the invention or a pharmaceutically acceptable salt of a compound of the invention. The amount of active compound in therapeutically useful compositions should be sufficient that a suitable dosage will be obtained.

When desired, formulations adapted to give sustained release of the active ingredient may be employed.

Examples of pharmaceutically acceptable salts include salts of pharmaceutically acceptable cations such as sodium, potassium, lithium, calcium, magnesium, ammonium and alkylammonium; acid addition salts of pharmaceutically acceptable inorganic acids such as hydrochloric, orthophosphoric, sulfuric, phosphoric, nitric, carbonic, boric, sulfamic and hydrobromic acids; or salts of pharmaceutically acceptable organic acids such as acetic, propionic, butyric, tartaric, maleic, hydroxymaleic, fumaric, citric, lactic, mucic, gluconic, benzoic, succinic, oxalic, phenylacetic, methanesulfonic, trihalomethanesulfonic, toluenesulfonic, benzenesulfonic, isethionic, salicylic, sulphanilic, aspartic, glutamic, edetic, stearic, palmitic, oleic, lauric, pantothenic, tannic, ascorbic, valeric and orotic acids. Salts of amine groups may also comprise quaternary ammonium salts in which the amino nitrogen atom carries a suitable organic group such as an alkyl, alkenyl, alkynyl or aralkyl moiety.

In some embodiments it may be preferable to formulate the 3-ethoxy-6-{2-[1-(6-methyl-pyridazin-3-yl)-piperidin-4-yl]-ethoxy}-benzo[d]isoxazole as its bis-dihydrogenphosphate and/or sulfate salt as disclosed in WO2009/143571 (the entire contents of which is incorporated herein by reference).

In some embodiments, the medicaments and pharmaceutical combinations for the use of the invention comprising 3-ethoxy-6-{2-[1-(6-methyl-pyridazin-3-yl)-piperidin-4-yl]-ethoxy}-benzo[d]isoxazole or a pharmaceutically acceptable salt thereof may be provided with instructions for use of the medicament or pharmaceutical combination.

The use of 3-ethoxy-6-{2-[1-(6-methyl-pyridazin-3-yl)-piperidin-4-yl]-ethoxy}-benzo[d]isoxazole or a pharmaceutically acceptable salt thereof in the stated applications, may further comprise the use of instructions.

The instructions may indicate a particular dosing regime, mode of administration, or otherwise, so as to indicate to the patient or physician, for example, how the medicament, combination, or method are to be applied to the intended application. For example the instructions may indicate how to use a medicament or combination, or perform a method, in the alleviation of symptoms of
asthma

Such instructions may indicate the separate, simultaneous or sequential administration of 3-ethoxy-6-{2-[1-(6-methyl-pyridazin-3-yl)-piperidin-4-yl]-ethoxy}-benzo[d]isoxazole or a pharmaceutically acceptable salt thereof and another medicament, such as an asthma medication.

As used herein, the term "subject" refers to any subject, preferably a vertebrate subject, and even more preferably a mammalian subject, for whom alleviation,
of symptoms is desired. Typically the subject is a human with pre-existing asthma

As used herein, "symptoms" of asthma refers to symptoms selected from the group consisting of reduced lung function (including reduced lung volume), coughing, wheezing, breathlessness and airway necrosis.

As used herein "alleviating" a symptom refers to the reduction of the severity or frequency of the symptom or both.

### EXAMPLES

The invention will now be described without limitation by reference to the examples which follow.

### EXAMPLE 1

### Phase II Double-Blind, Placebo-Controlled Study to determine the Prophylatic Efficacy of oral 3-ethoxy-6-{2-[1-(6-methyl-pyridazin-3-yl)-piperidin-4-yl]-ethoxy}-benzo[d]isoxazole in an experimental Rhinovirus Challenge Model

The design of the study was a placebo-controlled, double-blind, randomised, parallel group clinical trial.. The purpose of the study was to determine the efficacy, safety and pharmacokinetics of 10 days dosing with either 25mg, 100mg or 400mg 3-ethoxy-6-{2-[1-(6-methyl-pyridazin-3-yl)-piperidin-4-yl]-ethoxy}-benzo[d]isoxazole b.i.d. The efficacy of 3-ethoxy-6-{2-[1-(6-methyl-pyridazin-3-yl)-piperidin-4-yl]-ethoxy}-benzo[d]isoxazole was specifically assessed for preventing experimental HRV infection (virus challenge design).

Forty-one healthy male volunteers were enrolled in the study. Dose levels of the active were 25mg, 100mg and 400mg and the doses were administered as oral capsules of 25mg, 100mg and 200mg. Twice daily dosing of the active or placebo occurred on Day -2 to Day 6, followed by a single dose in the morning of Day 7. Subjects were inoculated with challenge virus (HRV39) on Day 0 at approximately 1 to 2 hours after the evening dose.

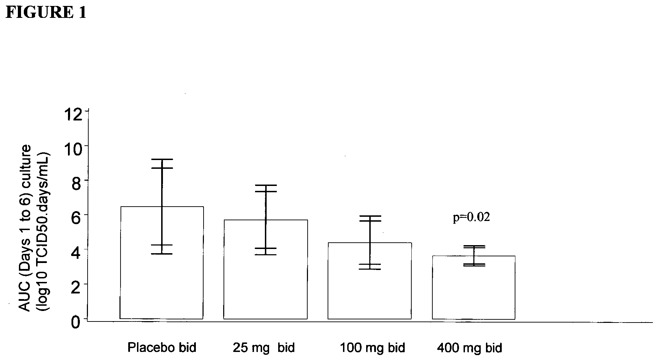

Nasal wash samples were taken for the assessment of viral load. Self- and physician-reported assessments of symptoms of upper respiratory tract illness were performed. Blood samples were taken to determine anti-HRV antibodies and for the analysis of plasma concentrations of the active and metabolites in serum and plasma. Mucus weight of nasal secretions was measured. The incidences of infection were compared between placebo and each dose level of the active using Fisher's Exact Test. The efficacy parameters derived from polymerase chain reaction (PCR) and culture data were compared between placebo and each dose level of the active using an analysis of variance model with a fixed effect for treatment. This study demonstrates that when used prophylatically, 3-Ethoxy-6-{2-[1-(6-methyl-pyridazin-3-yl)-piperidin-4-yl]-ethoxy}-benzo[d]isoxazole reduced the incidence of HRV39 infection in subjects in a dose-related manner. There was a dose-related difference to placebo in HRV viral load (AUC_{culture} and AUC_{PCR}) and in peak viral load between Days I to 6. These differences were statistically significant compared to placebo at the 400mg bid dose level. The culture results are presented Figure 1.

### EXAMPLE 2

### Human cell-derived tissue culture model for epithelial tissue of the human respiratory tract (EpiAirway™ system)

The EpiAirway™ system (MatTek Corporation, Ashland, MA) is a human cell-derived, fully differentiated, secretory, three-dimensional tissue culture model. The EpiAirway™ system consists of non-immortalized, human-derived tracheal / bronchial epithelial cells that have been cultured on microporous membranes in vitro. The primary cells form a pseudo-stratified, highly differentiated tissue culture model that closely resembles the epithelial tissue of the human respiratory tract. Histological cross-sections of the cultured tissue reveal a pseudo-stratified, mucociliary phenotype similar to a normal human bronchiole (Sheasgreen, J.K.M, et. al., The Toxicologist (1999) 48 (1-S):Astract#594). The epithelium is well-differentiated with functional cilia on the apical surface. The EpiAirway™ system also exhibits barrier properties similar to native tracheal / bronchial epithelium, including development of transepithelial electrical resistance, conferred by functional tight junctions.

MatTek's EpiAirway in vitro human tracheal/bronchial tissue equivalents can be produced from airway epithelium of diseased individuals (Hayden, P.J., Jackson, Jr., G.R., Bolmarcich, J., and Klausner, M. MatTek Corporation, Ashland, MA. Presented at American Thoracic Society Meeting, May (2009)). These tissues are excellent *in vitro* human models of asthma and COPD providing important unique attributes that animal models cannot provide, including the ability to address human individual variability and genetic factors, and a means to determine mechanisms of human virus elicitation of asthma and COPD exacerbations.

The microporous membranes onto which the primary cells are cultured can be found on inserts that are placed inside the wells of cell culture plates. The stratified cells are grown on the membranes at the air-liquid interface (ALI). Cell growth is maintained by addition of assay media to the well. At a specific point in the culturing process, all liquid is removed from the apical (top) surface of the tissue, and the tissues are then fed only through the basolateral (bottom) surface, which remains in contact with MatTek's proprietary assay media. Thus, the tissues are partially exposed to air. As rhinoviruses initiate infection by attachment to epithelial cells throughout the respiratory tract, virus can be added to the apical surface of the tissue to mimic exposure of the respiratory tract to HRV via the air. The antiviral activity of a compound can be assessed via addition to assay media that comes in contact with the basolateral surface of the tissue.

### Yield reduction assay performed using the EpiAirway™ System

The antiviral activity of 3-Ethoxy-6-{2-[1-(6-methyl-pyridazin-3-yl)-piperidin-4-yl]-ethoxy}-benzo[d]isoxazole free base against HRV serotype 14 in the EpiAirway™ system was measured using a virus yield reduction assay to determine the concentration of test compound that producers a 50% reduction in virus titre (EC₅₀).

The 96-well plate of the EpiAirway™ system (MatTek Corporation, Ashland, MA; catalogue number AIR-196-HTS) was equilibrated according to the manufacturer's protocol (EpiAirway HTS-96 use protocol). Briefly. 250 µL of the EpiAirway™ serum-free media (MatTek Corporation; catalogue number AIR-100-MM-ASY) was added to a feeder tray that allows the media to come into contact with the basolateral surface of the tissue. The plate was incubated for at least 18 hours at 37°C in a humidified 5% CO₂ atmosphere (Sanyo MCO-17AIC incubator; Quantum Scientific, Milton, Australia). Assay media was then removed from each well. Nine concentrations of 3-Ethoxy-6-{2-[1-(6-methyl-pyridazin-3-yl)-piperidin-4-yl]-ethoxy}-benzo[d]isoxazole free base ranging from 0.09 ng/mL to 1.14 µg/mL (0.24 nM to 2.98 µM), were prepared by serial dilution in assay media and 250 µL of each was then separately added to the basal surface of the membrane in each well. The negative control (250 µL assay media) was added to each of the negative control wells. Assay plates were incubated at 33°C in a humidified 5% CO₂ atmosphere overnight.

The apical surface of the tissue in the EpiAirway™ system was then inoculated with 1.8 x 10⁵ pfu per well (150 µL) of HRV 14. Assay plates were incubated at 33°C in a humidified 5% CO₂ atmosphere for eight hours, then 125 µL of media was removed from the apical surface of the tissue in each well. Samples were stored at -80°C prior to quantification by virus yield reduction assay.

### Quantification of virus titre from the in the EpiAirway™ System

HeLa Ohio cells were seeded in 96 well plates (Corning; catalogue number 3595) in 200 µL of assay media at a concentration of 1.0 x 10⁴ cells per well and incubated overnight at 37°C in a humidified 5% CO₂ atmosphere. After this incubation period, the cells were approximately 50% confluent.

Of the virus samples harvested from the apical surface of the tissue in each well of the EpiAirway™ system, 10 µL of each was diluted 1:100 in assay media. A volume of 100 µL of each dilution was added to each of seven wells in a new assay plate, and then these were serially diluted three-fold across the plate. resulting in a total of twelve different virus sample concentrations. Twelve wells contained assay media alone (i.e., no virus) and served as controls. Plates were incubated for five days at 33°C in a humidified 5% CO₂ atmosphere during which time cytopathic effects (CPE) was allowed to develop.

Virus-induced CPE of the cell monolayer was scored visually and the TCID₅₀ of the virus suspension was determined using the method of Reed-Muench (Reed, L.J. and Muench, H., Am. J. Hyg. (1938) 27: 493-7).

Resulting TCID₅₀ values for 3-Ethoxy-6-{2-[1-(6-methyl-pyridazin-3-yl)-piperidin-4-yl]-ethoxy}-benzo[d]isoxazole free base were expressed as a percentage of the negative control TCID₅₀ value.

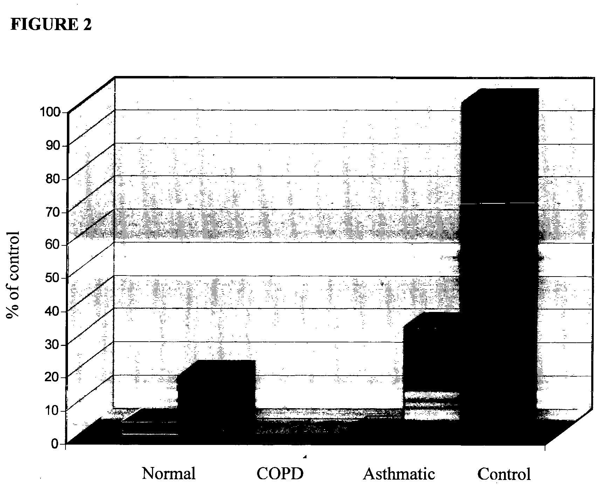

Figure 2 shows preliminary results (n=1) which indicate that 3-Ethoxy-6-{2-[1-(6-methyl-pyridazin-3-yl)-piperidin-4-yl]-ethoxy}-benzo[d]isoxazole free base is able to reduce infectious virus titres at concentrations of ranging from 200-1000 ng/mL in tissue derived from normal donors (570 ng/mL, 190 ng/mL) and.in those derived from asthmatic (1000 ng/mL, 330 ng/mL) and COPD (1000 ng/mL, 330 ng/mL) donors. EC₅₀ determination is in progress and values will be calculated from the percentage of the negative control value results by non-linear regression.

### EXAMPLE 3

### Phase II Multicentre, Randomised, Double-Blind, Placebo-Controlled, Parallel-Arm Study of two dose levels of 3-ethoxy-6-{2-[1-(6-methyl-pyridazin-3-yl)-piperidin-4-yl]-ethoxy}-benzo[d]isoxazole in Asthmatic Adults with symptomatic Human Rhinovirus Infection

The study population comprises a sample size of 229-400 subjects, male and female, aged 18-70 years, previously diagnosed with stable mild to moderate asthma at least 2 years prior to screening, pre-screened within 90 days prior to enrolment and presenting with symptoms of presumptive human rhinovirus (HRV). The subjects are randomised to receive placebo or one of two doses of 3-ethoxy-6-{2-[1-(6-methyl-pyridazin-3-yl)-piperidin-4-yl]-ethoxy}-benzo[d]isoxazole (1:1:1).

Asthma patients who have been identified and pre-screened will present to the clinic within 24 hours of symptomatic presumptive HRV infection onset. Eligible subjects are randomised to six days of treatment with placebo or total daily dosage of 800mg (400mg BID) of active ingredient with further clinic visits out to 28 days.

In this study the primary end point and rationale is based on the mean difference in the Wisconsin Upper Respiratory Symptom Survey-21 (WURSS-21) severity score (Walter, M.J., et. al., Eur Resp J (2008) 32:1548-1554) on Day 3 between the placebo and 3-ethoxy-6-{2-[1-(6-methyl-pyridazin-3-yl)-piperidin-4-yl]-ethoxy}-benzo[d]isoxazole. All subjects complete the WURSS-21 cold symptom scale daily for 14 days. Patients continue to maintain asthma medications according to their usual instructions and medication details such as dose and time of all dosing occasions is recorded on a diary card. The diary card is also used to record use of asthma reliever medications such as short acting beta agonists.

The secondary endpoints may include, but are not limited to, any one or more of the following: (1) Maximum mean percentage reduction in Peak Expiratory Flow (PEF) from Day 1 to Day 14 as a measure of lung function for virally induced asthma exacerbations; (2) the Forced Expiratory Volume in 1 second (FEV₁) recorded at clinic visits as a measure of lung function for virally induced asthma exacerbations; and (3) Asthma Control Questionnaire (ACQ-5) as a measure of the degree of control of the underlying asthma, and Asthma Quality of Life Questionnaire (AQLQS) scores for the impact of the current level of asthma on quality of life.

Nasal swabs are collected from all subjects for the virology studies. The key virology end points include the incidence of PCR positive samples for HRV at any time point, the incidence of positive viral culture over 2-4 days and AUC viral load from nasal swabs. The challenge study for 3-ethoxy-6-{2-[1-(6-methyl-pyridazin-3-yl)-piperidin-4-yl]-ethoxy}-benzo[d]isoxazole has demonstrated a clear dose response for viral load.

### EXAMPLE 4

### Capsule formulations

| **Component** | **Capsule 1 (25mg active) Quantity (%w/w)** | **Capsule 2 (100mg active) (%w/w)** | **Capsule 3 (200mg active) (%w/w)** |
|---|---|---|---|
| 3-ethoxy-6-{2-[1-(6-methyl-pyridazin-3-yl)-piperidin-4-yl]-ethoxy}-benzo[d]isoxazole *bis-*dihydrogenphosphate salt (1:2) | 5% | 28% | 68% |
| Glucose, anhydrous | 95% | 72% | 32% |

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The reference in this specification to any prior publication, or information derived from it, or to any matter which is know, is not, and should not be taken as an acknowledgement or admission or any form of suggestion that that prior publication, or information derived from it, or known matter forms part of the common general knowledge in the field of endeavour to which this specification relates.

## Claims

1. 3-Ethoxy-6-{2-[1-(6-methyl-pyridazin-3-yl)-piperidin-4-yl]-ethoxy}-benzo[d]isoxazole or a pharmaceutically acceptable salt thereof for use in the alleviation of symptoms of asthma in a subject having a HRV infection wherein the symptoms of asthma are selected from the group consisting of reduced lung function, reduced lung volume, coughing, wheezing, breathlessness and airway necrosis and wherein 3-ethoxy-6-{2-[1-(6-methyl-pyridazin-3-yl)-piperidin-4-yl]-ethoxy}-benzo[d]isoxazole or the pharmaceutically acceptable salt thereof is formulated for oral administration.

2. 3-Ethoxy-6-{2-[1-(6-methyl-pyridazin-3-yl)-piperidin-4-yl]-ethoxy}-benzo[d]isoxazole or a pharmaceutically acceptable salt thereof for use according to claim 1, wherein the incidence of exacerbations of the symptoms of asthma is reduced.

3. 3-Ethoxy-6-{2-[1-(6-methyl-pyridazin-3-yl)-piperidin-4-yl]-ethoxy}-benzo[d]isoxazole or a pharmaceutically acceptable salt thereof for use according to claim 1 or 2 wherein the orally administered 3-ethoxy-6-{2-[1-(6-methyl-pyridazin-3-yl)-piperidin-4-yl]-ethoxy}-benzo[d]isoxazole or the pharmaceutically acceptable salt thereof is in a solid form or a liquid form.

4. 3-Ethoxy-6-{2-[1-(6-methyl-pyridazin-3-yl)-piperidin-4-yl]-ethoxy}-benzo[d]isoxazole or a pharmaceutically acceptable salt thereof for use according to claim 3 wherein the solid form is a tablet or capsule.

5. 3-Ethoxy-6-{2-[1-(6-methyl-pyridazin-3-yl)-piperidin-4-yl]-ethoxy}-benzo[d]isoxazole or a pharmaceutically acceptable salt thereof for use according to any one of claims 1 to 4 wherein the symptom of asthma is reduced lung function.

6. 3-Ethoxy-6-{2-[1-(6-methyl-pyridazin-3-yl)-piperidin-4-yl]-ethoxy}-benzo[d]isoxazole or a pharmaceutically acceptable salt thereof for use according to claim 5 wherein the reduced lung function is reduced lung volume.

7. 3-Ethoxy-6-{2-[1-(6-methyl-pyridazin-3-yl)-piperidin-4-yl]-ethoxy}-benzo[d]isoxazole or a pharmaceutically acceptable salt thereof for use according to claim 5 wherein the reduced lung function is measured by maximum mean percentage reduction in Peak Expiratory Flow (PEF).

8. 3-Ethoxy-6-{2-[1-(6-methyl-pyridazin-3-yl)-piperidin-4-yl]-ethoxy}-benzo[d]isoxazole or a pharmaceutically acceptable salt thereof for use according to claim 5 wherein the reduced lung function is measured by the Forced Expiratory Volume in 1 second (FEV1).

9. 3-Ethoxy-6-{2-[1-(6-methyl-pyridazin-3-yl)-piperidin-4-yl]-ethoxy}-benzo[d]isoxazole or a pharmaceutically acceptable salt thereof for use according to claim 5 wherein the reduced lung function is measured by an Asthma Controlled Questionnaire (ACQ-5) and Asthma Quality of Life Questionnaire (AQLS).

10. 3-Ethoxy-6-{2-[1-(6-methyl-pyridazin-3-yl)-piperidin-4-yl]-ethoxy}-benzo[d]isoxazole or a pharmaceutically acceptable salt thereof for use according to any one of claims 1 to 9 wherein the 3-ethoxy-6-{2-[1-(6-methyl-pyridazin-3-yl)-piperidin-4-yl]-ethoxy}-benzo[d]isoxazole or the pharmaceutically acceptable salt thereof is formulated in a dosage amount of from 1 mg to 800 mg per day.

11. 3-Ethoxy-6-{2-[1-(6-methyl-pyridazin-3-yl)-piperidin-4-yl]-ethoxy}-benzo[d]isoxazole or a pharmaceutically acceptable salt thereof for use according to any one of claims 1 to 10 wherein the subject has a predisposition to or pre-existing asthma.

## Patentansprüche

1. 3-Ethoxy-6-{2-[1-(6-methyl-pyridazin-3-yl)-piperidin-4-yl]-ethoxy}-benzo[d]isoxazol oder ein pharmazeutisch annehmbares Salz davon zur Verwendung zur Linderung von Asthmasymptomen bei einer Person mit einer HRV-Infektion, wobei die Asthmasymptome ausgewählt sind aus der Gruppe bestehend aus verringerter Lungenfunktion, verringertem Lungenvolumen, Husten, Keuchen, Atemnot und Atemwegsnekrose und wobei 3-Ethoxy-6-{2-[1-(6-methyl-pyridazin-3-yl)-piperidin-4-yl]-ethoxy}-benzo[d]isoxazol oder das pharmazeutisch annehmbare Salz davon für die orale Verabreichung formuliert ist.

2. 3-Ethoxy-6-{2-[1-(6-methyl-pyridazin-3-yl)-piperidin-4-yl]-ethoxy}-benzo[d]isoxazol oder ein pharmazeutisch annehmbares Salz davon zur Verwendung nach Anspruch 1, wobei die Häufigkeit von Verschlimmerungen der Asthmasymptome verringert wird.

3. 3-Ethoxy-6-{2-[1-(6-methyl-pyridazin-3-yl)-piperidin-4-yl]-ethoxy}-benzo[d]isoxazol oder ein pharmazeutisch annehmbares Salz davon zur Verwendung nach Anspruch 1 oder 2, wobei das oral verabreichte 3-Ethoxy-6-{2-[1-(6-methyl-pyridazin-3-yl)-piperidin-4-yl]-ethoxy}-benzo[d]isoxazol oder das pharmazeutisch annehmbare Salz davon in einer festen Form oder einer flüssigen Form vorliegt.

4. 3-Ethoxy-6-{2-[1-(6-methyl-pyridazin-3-yl)-piperidin-4-yl]-ethoxy}-benzo[d]isoxazol oder ein pharmazeutisch annehmbares Salz davon zur Verwendung nach Anspruch 3, wobei die feste Form eine Tablette oder Kapsel ist.

5. 3-Ethoxy-6-{2-[1-(6-methyl-pyridazin-3-yl)-piperidin-4-yl]-ethoxy}-benzo[d]isoxazol oder ein pharmazeutisch annehmbares Salz davon zur Verwendung nach einem der Ansprüche 1 bis 4, wobei das Asthmasymptom eine verringerte Lungenfunktion ist.

6. 3-Ethoxy-6-{2-[1-(6-methyl-pyridazin-3-yl)-piperidin-4-yl]-ethoxy}-benzo[d]isoxazol oder ein pharmazeutisch annehmbares Salz davon zur Verwendung nach Anspruch 5, wobei die verringerte Lungenfunktion ein verringertes Lungenvolumen ist.

7. 3-Ethoxy-6-{2-[1-(6-methyl-pyridazin-3-yl)-piperidin-4-yl]-ethoxy}-benzo[d]isoxazol oder ein pharmazeutisch annehmbares Salz davon zur Verwendung nach Anspruch 5, wobei die verringerte Lungenfunktion gemessen ist als maximale mittlere prozentuale Verringerung des exspiratorischen Spitzenflusses (Peak Expiratory Flow, PEF).

8. 3-Ethoxy-6-{2-[1-(6-methyl-pyridazin-3-yl)-piperidin-4-yl]-ethoxy}-benzo[d]isoxazol oder ein pharmazeutisch annehmbares Salz davon zur Verwendung nach Anspruch 5, wobei die verringerte Lungenfunktion gemessen ist als das forcierte Ausatemvolumen in 1 Sekunde (Forced Expiratory Volume in 1 second, FEV1).

9. 3-Ethoxy-6-{2-[1-(6-methyl-pyridazin-3-yl)-piperidin-4-yl]-ethoxy}-benzo[d]isoxazol oder ein pharmazeutisch annehmbares Salz davon zur Verwendung nach Anspruch 5, wobei die verringerte Lungenfunktion über einen Asthmakontrolle-Fragebogen (Asthma Controlled Questionnaire, ACQ-5) und Asthma-Lebensqualität-Fragebogen (Asthma Quality of Life Questionnaire, AQLS) gemessen ist.

10. 3-Ethoxy-6-{2-[1-(6-methyl-pyridazin-3-yl)-piperidin-4-yl]-ethoxy}-benzo[d]isoxazol oder ein pharmazeutisch annehmbares Salz davon zur Verwendung nach einem der Ansprüche 1 bis 9, wobei das 3-Ethoxy-6-{2-[1-(6-methyl-pyridazin-3-yl)-piperidin-4-yl]-ethoxy}-benzo[d]isoxazol oder das pharmazeutisch annehmbare Salz davon in einer Dosierungsmenge von 1 mg bis 800 mg pro Tag formuliert ist.

11. 3-Ethoxy-6-{2-[1-(6-methyl-pyridazin-3-yl)-piperidin-4-yl]-ethoxy}-benzo[d]isoxazol oder ein pharmazeutisch annehmbares Salz davon zur Verwendung nach einem der Ansprüche 1 bis 10, wobei die Person eine Prädisposition für Asthma oder vorbestehendes Asthma hat.

## Revendications

1. 3-Ethoxy-6-{2-[1-(6-méthyl-pyridazin-3-yl)-pipéridin-4-yl]-éthoxy}-benzo[d]-isoxazole ou l'un de ses sels pharmaceutiquement acceptables pour une utilisation dans l'atténuation des symptômes de l'asthme chez un sujet souffrant d'une infection à RVH où les symptômes de l'asthme sont choisis dans le groupe constitué de la fonction pulmonaire réduite, le volume pulmonaire réduit, la toux, les sifflements, l'essoufflement et la nécrose des voies respiratoires et où le 3-éthoxy-6-{2-[1-(6-méthyl-pyridazin-3-yl)-pipéridin-4-yl]-éthoxy}-benzo[d]isoxazole ou l'un de ses sels pharmaceutiquement acceptables est formulé pour une administration orale.

2. 3-Ethoxy-6-{2-[1-(6-méthyl-pyridazin-3-yl)-pipéridin-4-yl]-éthoxy}-benzo[d]-isoxazole ou l'un de ses sels pharmaceutiquement acceptables pour une utilisation selon la revendication 1, où l'incidence des exacerbations des symptômes de l'asthme est réduite.

3. 3-Ethoxy-6-{2-[1-(6-méthyl-pyridazin-3-yl)-pipéridin-4-yl]-éthoxy}-benzo[d]-isoxazole ou l'un de ses sels pharmaceutiquement acceptables pour une utilisation selon la revendication 1 ou 2 où le 3-éthoxy-6-{2-[1-(6-méthyl-pyridazin-3-yl)-pipéridin-4-yl]-éthoxy}-benzo[d]isoxazole ou l'un de ses sels pharmaceutiquement acceptables administré par voie orale est sous forme solide ou sous forme liquide.

4. 3-Ethoxy-6-{2-[1-(6-méthyl-pyridazin-3-yl)-pipéridin-4-yl]-éthoxy}-benzo[d]-isoxazole ou l'un de ses sels pharmaceutiquement acceptables pour une utilisation selon la revendication 3 où la forme solide est un comprimé ou une capsule.

5. 3-Ethoxy-6-{2-[1-(6-méthyl-pyridazin-3-yl)-pipéridin-4-yl]-éthoxy}-benzo[d]-isoxazole ou l'un de ses sels pharmaceutiquement acceptables pour une utilisation selon l'une quelconque des revendications 1 à 4 où le symptôme de l'asthme est la fonction pulmonaire réduite.

6. 3-Ethoxy-6-{2-[1-(6-méthyl-pyridazin-3-yl)-pipéridin-4-yl]-éthoxy}-benzo[d]-isoxazole ou l'un de ses sels pharmaceutiquement acceptables pour une utilisation selon la revendication 5 où la fonction pulmonaire réduite est le volume pulmonaire réduit.

7. 3-Ethoxy-6-{2-[1-(6-méthyl-pyridazin-3-yl)-pipéridin-4-yl]-éthoxy}-benzo[d]-isoxazole ou l'un de ses sels pharmaceutiquement acceptables pour une utilisation selon la revendication 5 où la fonction pulmonaire réduite est mesurée par la réduction moyenne maximale en pourcentage du débit expiratoire maximal (DEM).

8. 3-Ethoxy-6-{2-[1-(6-méthyl-pyridazin-3-yl)-pipéridin-4-yl]-éthoxy}-benzo[d]-isoxazole ou l'un de ses sels pharmaceutiquement acceptables pour une utilisation selon la revendication 5 où la fonction pulmonaire réduite est mesurée par le volume expiratoire forcé en 1 seconde (VEMS).

9. 3-Ethoxy-6-{2-[1-(6-méthyl-pyridazin-3-yl)-pipéridin-4-yl]-éthoxy}-benzo[d]-isoxazole ou l'un de ses sels pharmaceutiquement acceptables pour une utilisation selon la revendication 5 où la fonction pulmonaire réduite est mesurée par un questionnaire de contrôle de l'asthme (ACQ-5) et un questionnaire de qualité de vie de l'asthme (AQLS).

10. 3-Ethoxy-6-{2-[1-(6-méthyl-pyridazin-3-yl)-pipéridin-4-yl]-éthoxy}-benzo[d]-isoxazole ou l'un de ses sels pharmaceutiquement acceptables pour une utilisation selon l'une quelconque des revendications 1 à 9 où le 3-éthoxy-6-{2-[1-(6-méthyl-pyridazin-3-yl)-pipéridin-4-yl]-éthoxy}-benzo[d]isoxazole ou l'un de ses sels pharmaceutiquement acceptables est formulé dans une quantité posologique de 1 mg à 800 mg par jour.

11. 3-Ethoxy-6-{2-[1-(6-méthyl-pyridazin-3-yl)-pipéridin-4-yl]-éthoxy}-benzo[d]-isoxazole ou l'un de ses sels pharmaceutiquement acceptables pour une utilisation selon l'une quelconque des revendications 1 à 10 où le sujet a une prédisposition à ou souffre d'un asthme préexistant.
